(19) Europäisches Patentamt
European Patent Office
Office européen des brevets

(11) Publication number: **0 248 824**
**B1**

(12) **EUROPEAN PATENT SPECIFICATION**

(45) Date of publication of the patent specification: **17.01.90**

(51) Int. Cl.⁴: **A 61 K 31/165**, A 61 K 31/40, C 07 D 295/12

(21) Application number: **86906654.8**

(22) Date of filing: **21.10.86**

(86) International application number: **PCT/US 86/02228**

(87) International publication number: **WO 87/02584 (07.05.87 Gazette 87/10)**

(54) **Use of CIS-N-(2 Aminocycloaliphatic) benzene acetamide and Benzamide for the manufacture of anticonvulsants.**

(30) Priority: **25.10.85 US 791311**

(43) Date of publication of application: **16.12.87 Bulletin 87/51**

(45) Publication of the grant of the patent: **17.01.90 Bulletin 90/03**

(84) Designated Contracting States: **AT BE CH DE ES FR GB GR IT LI LU NL SE**

(73) Proprietor: **THE UPJOHN COMPANY**
**301 Henrietta Street**
**Kalamazoo, Michigan 49001 (US)**

(72) Inventor: **SZMUSZKOVICZ, Jacob**
**3420 Pinegrove Lane**
**Kalamazoo, MI 49001 (US)**
Inventor: **VON VOIGTLANDER, Philip, F.**
**1 South Lake Doster Drive**
**Plainwell, MI 49080 (US)**

(74) Representative: **Perry, Robert Edward**
**GILL JENNINGS & EVERY 53-64 Chancery Lane**
**London WC2A 1HN (GB)**

**EP 0 248 824 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBERGRAF, STOCKHOLM 1990

## Description

This invention relates to the use of certain N-(2-amino cycloaliphatic)benzeneacetamide and -benzamide compounds as Central Nervous System (CNS) anti-seizure, e.g. , anti-convulsant, drugs in valuable warm-blooded animals, including humans.

## Background of the Invention

Szmuszkovicz U.S. Patent No. 4 098 904 discloses some N-(2-amino cycloaliphatic)benzamide compounds, e.g., N-methyl-N-[2-(1-pyrrolidinyl)cyclohexyl]-3,4-dichlorobenzamide, and N-methyl-N-[2-(N',N'-dimethylamino)cyclohexyl]-4-bromobenzamide, and salts and hydrates thereof as analgesic (pain reducing) drug compounds.

Szmuszkovicz U.S. Patent No. 4 145 435 discloses some 2-aminocycloaliphatic alkanoyl amide compounds, e.g., N-methyl-N-[2-(N',N'-dimethylamino)-cyclohexyl]-2-(4-bromophenyl)acetamide and trans-2-(3,4dichlorophenyl-N-methyl-N-[2-(1-pyrrolidinyl)cyclohexyl]acetamide, and their pharmaceutically acceptable salts as analgesic compounds.

In contrast to the disclosure in those Szmuszkovicz '904 and '435 patents, the compounds of this invention have little or no significant analgesic activity ($ED_{50}$ >50), but have been found to possess significant CNS seizure preventing or blocking activity in standard laboratory animal tests.

In *Neuroscience Abstracts*, *10*, page 408 (October, 1984), F.C.Tortella et al. report the Seizure-specific, Dose- And Time- Dependant Anti-convulsant Profile of Upjohn Compound, Trans-(±)-3,4-dichloro-N-methyl-N-[2-(1-pyrrolidinyl)cyclohexyl]benzeneacetamide by an electroshock test method. This compound is described and claimed in the above Szmuszkovicz '435 patent; however, as indicated hereinabove, this compound is an analgesically active compound.

Recent pharmacology articles that may be of interest as background are:

*J. Med. Chem.*, 1984, *27*, 779 - 782, "Anticonvulsant... 4-Aminobenzamides" by C. Randall Clark et al.,

*Science*, *226*. 16 November 1984, pp. 850 - 852, "Blockade... Against Ischemic Damage in the Brain" by R.P. Simon et al., and

*Neuropharmacology*, *23*, No. 3, pp. 367 - 371 (1984), "The Anti-convulsant... Electroshock Seizures in Rats" by E.F. Berman et al..

## Summary of the Invention

Compounds of formula I and their pharmaceutically-acceptable salts have a new use, i.e. for the prevention or treatment of central nervous system seizures. The compounds have only minimal or insignificant analgesic activity in standard laboratory animal tests.

With respect to formula I, the filled circles indicate that the two nitrogen atoms are in the cis orientation;

n is 1 or 2 (indicating a cyclopentyl or cyclohexyl ring, respectively);
m is 0 or 1 (indicating that the compounds are benzamides or benzeneacetamides, respectively);
R is hydrogen or $C_{1-3}$ alkyl;
either $R_1$ and $R_2$ are the same or different $C_{1-3}$ alkyl groups, or $NR_1R_2$ is 1-pyrrolidinyl; and
either X and Y are independently selected from H, F, Br and Cl, or X is H and Y is $CF_3$.

Novel compounds of the invention are
cis-3,4-dichloro-N-[2-(1-pyrrolidinyl)cyclopentyl]benzeneacetamide,
cis-3,4-dichloro-N-methyl-N-[2-(1-pyrrolidinyl)cyclopentyl]benzamide,
cis-3,4-dichloro-N-[2-(1-pyrrolidinyl)cyclohexyl]benzamide,
cis-3,4-dichloro-N-methyl-N-[2-(1-pyrrolidinyl)cyclohexyl]benzamide,
and pharmaceutically-acceptable salts thereof.

## Detailed Description of the Invention

The group of compounds described herein have been found to be effective anti-convulsant drug agents in standard animal tests. These compounds have been shown to be effective antagonists of electroshock seizures in mice. They will be useful in the treatment of grand mal and other seizure disorders in man as well as in commercially important and pet animals.

These compounds are active in the CNS electroshock animal test but they did not at similar dosage ranges block chemically induced (pentylenetetrazol or bicucullin) seizures in the test animals suggesting that these compounds have a CNS anti-seizure utility similar to that of the known drug phenytoin (The Merck Index. 10th. Edition, page 1054, item 7204). Phenytoin is useful in the treatment of grand mal, focal and psychomotor seizures (see Goodman and Gilman, The Pharmacological Basis of Therapeutics). However, phenytoin and structurally related anti-convulsant drugs are known to cause a number of side effects including hyperplasia of the gums, cerebellar degeneration, gastric distress and serious skin rashes.

In contrast, the compounds of this invention, while being anti-seizure drugs, e.g., anti-convulsant drugs, at reasonable dosages, have little or no significant analgesic activity, and it ts also hoped that these cis-compounds will not show some of the other above-listed side effects as well. The anti-convulsant potency of these cis-aminoamide compounds in standard laboratory animal tests indicate that these compounds will be useful for preventing or treating CNS seizure disorders in warm-blooded animals such as cats, dogs, horses, as well as humans at dosage ranges of approximately 0.1 to 100 mg/kg of body weight/day via the oral or parenteral routes until the seizure threat or attack subsides.

The compounds of this invention, of formula I in the General Formulas hereinbelow, are cis-N-[(2-aminocycloaliphatic)benzeneacetamide and -benzamide compounds where the two large filled circle positions in the ring are intended to depict the cis orientation of the two nitrogen groups relative to each other and the ring.

n is 1 or 2, to indicate a cyclopentyl or cyclohexyl ring;

m is 0 or 1, to indicate that the compounds are benzeneacetamides (with the $-CH_2-$) or benzamides (without the $-CH_2-$),

R is hydrogen or $C_1$ to $C_3$-alkyl, e.g., methyl, ethyl, isopropyl or n-propyl, preferably methyl, when R is $C_1$ to $C_3$-alkyl,

$R_1$ and $R_2$ taken separately, are independently $C_1$ to $C_3$ -alkyl, as defined above, or

$R_1$ and $R_2$ can be taken together with the nitrogen to which they are bonded to complete a pyrrolidine ring,

X and Y are each hydrogen, a halogen having an atomic number of from 9 to 35, that is, fluorine, chlorine or bromine, trifluoromethyl, preferably chlorine, in the 3- and the 4-position or bromine in the 4-position, or trifluoromethyl in the 4-position, and when

X and Y are each hydrogen, R is preferably hydrogen, m is 0, n is 1 or 2, and $R_1$ and $R_2$ are each $C_1$ to $C_3$ -alkyl, preferably methyl, or a pharmaceutically acceptable salt thereof.

Acid addition salts of these amino-amides (I) can be prepared by reacting a Formula I free base with a stoichiometric amount of an acid, such as hydrogen chloride, hydrogen bromide, hydrogen iodide, sulfuric acid, phosphoric acid, acetic acid, lactic acid, citric acid, succinic acid, benzoic acid, salicyclic acid, pamoic acid, cyclohexanesulfamic acid, methanesulfonic, naphthalenesulfonic, p-toluenesulfonic, maleic, fumaric or oxalic acids. The reaction can be carried out in aqueous or organic liquid solvent non-aqueous media such as diethyl ether or ethyl acetate. Non-aqueous media are preferred.

On occasion the compounds (I) or their acid addition salts in their crystalline state are isolated as solvates, e.g., with a discrete quantity of solvent, e.g., water or methanol associated physically, and thus not affecting the chemical entity per se.

The compositions containing a Formula I compound as an active ingredient in a pharmaceutical carrier are useful in pharmaceutical dosage unit forms of the Formula I compounds for systemic administration (oral, rectal and parenteral (including intravenous, intramuscular and intra-arterial) administration form) for treating warm-blooded animal patients, cats, dogs, horses and other commercially valuable animals, and human patients suspected of being susceptible to or to stop CNS seizures, such as convulsions, grand mal, petit mal and other seizure disorders. The term "dosage unit form" as used in this specification refers to physically discrete units suitable as unitary dosages for mammalian subjects, each unit containing a predetermined quantity of the essential active ingredient compound of this invention calculated to produce the desired effect in combination with the required pharmaceutical means which adapt the said ingredient for topical or systemic administration The specifications for the novel dosage unit forms of this invention are indicated by and directly dependent on the physical characteristics of the essential active ingredient and the particular effect to be achieved in view of the limitations inherent in the art of compounding such an essential active material for beneficial effects in humans and animals. Examples of suitable dosage unit forms in accordance with this invention are tablets, capsules, orally administered liquid preparations in suitable liquid vehicles, sterile preparations in suitable liquid vehicles for intramuscular and intravenous administration, suppositories, and sterile dry preparations for the extemporaneous preparation of sterile injectable preparations in a suitable liquid vehicle. Suitable solid diluents or carriers for the solid oral pharmaceutical dosage unit forms are selected from the group consisting of lipids, carbohydrates, proteins and mineral solids, for example, starch, sucrose, lactose, kaolin, dicalcium phosphate, gelatin, acacia, corn syrup, corn starch or talc. Capsules both hard and soft are filled with composition of these amino amide ingredients in combination with suitable diluents and excipients, for example, edible oils, talc, calcium carbonate and or calcium stearate. Liquid preparations for oral administration are prepared in water or aqueous vehicles which advantageously contain suspending agents, for example, methylcellulose, acacia, polyvinylpyrrolidone or polyvinyl alcohol. In the case of injectable forms, the injectable formulation must be sterile and must be fluid to the extent that easy syringeability exists. Such preparations must be stable under the conditions of manufacture and storage, and ordinarily contain in addition to the basic solvent or suspending liquid, preservatives in the nature of bacteriostatic and fungistatic agents, for example, parabens, chlorobutanol, benzyl alcohol, phenol or thimerosal. In many cases it is preferable to include osmotically active agents, for example, sugars or sodium chloride in isotonic concentrations. Carriers and vehicles include vegetable oils, ethanol, polyols, for example, glycerol, propylene glycol or liquid polyethylene glycol. Any solid preparations for subsequent extemporaneous preparation of sterile injectable preparations are sterilized, preferably by exposure to a sterilizing gas, for example ethylene oxide. The aforesaid carriers, vehicles, diluents, excipients, preservatives, isotonic agents constitute the pharmaceutical means which adapt the preparations for systemic administration.

The pharmaceutical dosage unit forms are prepared in accordance with the preceding general description to provide from about 1.0 to about 700 mg of the essential active ingredient per dosage unit form, which as aforesaid may be in the form of a solid, semi-solid, topical, oral, or rectal preparation, a liquid oral preparation, an injectable preparation, including liquid preparations and solid dry preparations for extemporaneous reconstitution to a liquid injectable preparation. The amount of the essential active ingredient provided in the pharmaceutical dosage unit forms is that amount sufficient to obtain a reduction in the CNS seizure effects and a return to more normal CNS stability, or to prevent the occurrence of CNS seizures in a patient who is suspected to be subject to such seizure. Expressed otherwise an amount of the essential active ingredient is provided to a recipient within a range from about 0.1 mg per kg to about 100 mg per kg of body weight of the recipient. Preferred dosages for most applications are 1.0 to 10.0 mg per kg of body weight.

The useful pharmaceutical dosage unit forms of these compounds in pharmaceutical formulations is preferably adapted for systemic administration to obtain anti-convulsant effects comprising an effective, non-toxic amount of a compound according to Formula I or as its pharmacologically acceptable salt.

These specific cis-amino-amide compounds have an advantage, to a greater or lesser extent, depending upon the particular compound of having significant CNS antiseizure properties, while having little or no significant analgesic activity (analgesic $ED_{50}$ >50 in the HCl writhing test). This combination of properties should prove beneficial and advantageous where the doctor would prefer to treat the patient for the single CNS seizure disorder without need to worry about any significant analgesic side effects.

The cis-amino-amide compounds involved in this invention can be made by procedures described in the Szmuszkovicz U.S. Patents 4 098 904 and 4 145 435, *supra*, using the cis-1,2-diamines referred to in those two patents, and the appropriate acylating form of the selected benzeneacetic acid, or -acetyl halide, or the selected benzoic acid, benzoyl halide, as described generally therein. Described hereinbelow are procedures used to prepare the compounds described and exemplified herein. Scheme I and II attached hereto, show a typical procedure for preparing the compounds involved in this invention.

Scheme I outlines in general chemical symbol form a process for preparing cis-diamine starting materials, which can be used to make the cis-2-amino-cycloaliphatic-benzeneacetamide and -benzamide compounds, which are used according to the invention described herein.

In general, the alkyl cycloalkanone carboxylate ester starting material (II), which can be, e.g., the pure methyl, ethyl or propyl esters or the commercially available mixed esters, and the selected $HNR_1R_2$ amine, e.g., pyrrolidine, dimethylamine, diethylamine, di-n-propylamine, di-ispropylamine, or a mixed amine such as N-methyl-Nethylamine, are mixed and reacted in a selected solvent such as benzene, toluene or heptane while heating under reflux conditions to remove water by-product from the reaction mixture to form the respective alkyl 2-aminocycloalk-1-enylcarboxylate ester (III). Alternatively, the alkyl 2-amino-cycloalk-1-enylcarboxylate ester (III) can be prepared from a 1-cycloalkenylamine (IV) and a carboxylation agent.

The resulting 2-aminocycloalk-1-enylcarboxylate ester (III) can then be reduced, e.g., by hydrogenating the reaction mixture containing the ester (III) in the presence of a hydrogenation catalyst such as platinum oxide to form the saturated cis 2-aminocycloaliphatic ester (V).

The cis- saturated ring amino-ester (V) can then be treated with hydrazine in the presence of an appropriate solvent, such as a $C_1$ to $C_4$-alkanol, under reflux conditions to form the cis-amino-hydrazide (VI). The hydrazide (VI) can be subjected to a nitrosation-Curtius rearrangement reaction to form the cis-2-aminocycloalkyl primary amine (VII) which can be used directly to make end product compounds of structure I herein where R is hydrogen.

For compounds where R is to be a $C_1$ to $C_3$-alkyl, the cis-2-aminocycloalkylamine VII is further acylated with an alkyl formate (to form an R equals methyl compound, or with an acetyl halide or propionyl halide, e.g., acetyl chloride or propionyl chloride, in the presence of a tertiary amine hydrogen halide scavenger to form the cis-N-(2-aminocycloalkyl) $C_1$ to $C_3$-alkanoylamide (VIII), where $R_3$ is hydrogen or $C_1$ to $C_2$-alkyl. This resulting amino-amide (VIII) can then be subjected to carbonyl group reduction, e.g., with lithium aluminum hydride, to convert the $R_3$-C(O)- group on the nitrogen in the 1-position of the cycloalkyl ring to the defined R equals $C_1$ to $C_3$-alkyl group, and to form cis-diamine (IX).

Scheme II describes in chemical symbol form an amine nitrogen acylation process that can be used to make the cis N-[2-(aminocycloaliphatic)-N-benzeneacetamide (m = 1) and -benzamide (m = 0) compounds which can then be recovered from their reaction mixtures, and used according to this invention or converted to appropriate acid addition salt form for recovery from its reaction mixture, and then re-formed, if desired or necessary, into an appropriate, pharmaceutically acceptable acid addition salt form for compounding into appropriate marketing and dosage unit forms.

Here, in Scheme II the cis-cycloaliphatic diamine (IX) can be acylated, by known procedures, e.g., by reaction with a benzeneacetyl halide or benzoyl halide (X), in the presence of a tertiary amine hydrogen halide scavenger, such as triethylamine or N-N-dimethylaniline to form the N-[2-(aminocycloalkyl)-N-(H- or alkyl)benzeneacetamine or-benzamide. These latter compounds can then be processed as indicated herein to put the compounds in pharmaceutically acceptable useful form.

Preparation CIS-2-PYRROLIDINCYCLOHEXYLAMINE

This preparation exemplifies how cis-1,2-cyclohexanediamines were prepared, for use in preparing the cis-amino-amide compounds which are involved in the invention described and claimed herein.

A. Ethyl cis-2-pyrrolidinylcyclohexanecarboxylate

A solution of 164.4 g (1.0 mole) ethyl 2-cyclohexanone carboxylate [also named ethyl 2-oxocyclohexanoate], which also contained about 40 percent of the corresponding methyl ester, and 71.1 g (1.0 mole) of pyrrolidine in 700 ml of benzene was refluxed overnight with azeotropic distillation of water therefrom (18 ml $H_2O$ collected) to form the ethyl and methyl 2-(1-pyrrolidinyl)-1-cyclohexen-1-ylcarboxylate mixed ester. Then 10 g of platinum oxide was added to this resulting reaction mixture, and the mixture was hydrogenated at 30 psig. until uptake of hydrogen ceased. About 0.95 mole of hydrogen was consumed. The resulting hydrogenated reaction mixture contained the cis-ethyl and methyl mixed 2-(1-pyrrolidinyl)cyclohexanecarboxylate esters. This reaction mixture was filtered through a filter aid (CELITE®) to remove the platinum catalyst residue. The filtered liquid was then extracted with 200 ml of 6N hydrochloric acid. The resulting aqueous layer was washed with ether, made basic with 15 percent sodium hydroxide and again extracted with ether. The original organic layer and the ether wash were combined as one organic layer and washed with saturated sodium chloride in water solution, dried ($MgSO_4$) and evaporated to remove solvents. Distillation of the residue gave the cis-2-(1-pyrrolidinyl)-cyclohexanecarboxylate, mixed methyl and ethyl esters, 143.5 g (70 percent yield, b.p. 95° - 105°C/0.5 mm (Hg).

This cis-amino-cyclohexanecarboxylate mixed ester material was used in the next step of the process without further purification.

B. Cis-2-Pyrrolidinylcyclohexanecarboxhydrazide, and its hydrochloride.

A solution of 143.5 g (0.65 mole) of the named amino-mixed ester material from part A hereinabove and 162.5 g. (3.25 moles) of hydrazine hydrate in 1300 ml of absolute ethanol was stirred at room temperature for 24 hours, and then refluxed for 72 hours to form the 2-(1-pyrrolidinyl)cyclohexanecarboxhydrazide. The solvent was largely removed by evaporation. The residue was diluted with 1500 ml of methylene chloride, washed twice with 150 ml portions of saturated aqueous sodium chloride solution, dried ($MgSO_4$) and evaporated to remove most of the methylene chloride.

The 2-(1-pyrrolidinyl)cyclohexanecarboxhydrazide hydrochloride salt was prepared by treatment of this hydrazide residue with hydrogen chloride in ether solution and was recrystallized from a methanol/ethel solvent mixture to give the cis-2-(1-pyrrolidinyl)cyclohexanecarboxhydrazide hydrochloride, 126.8 g, (69 percent yield), m.p. 222° - 224°C. The UV, mass spectrum, IR and NMR spectra were consistent with this named compound.

Anal. Calcd. for $C_{11}H_{21}N_3O \cdot 2HCl$:

| | |
|---|---|
| % Calcd: | C, 46.48; H, 8.16, N, 14.78; Cl, 24.95 |
| Found: | C, 45.49; H, 8.22; N, 15.20, Cl, 25.50 |

C. Cis-2-(1-Pyrrolidinyl)cyclohexaneamine

To a solution of 53.4 g (0.188 mole) of the 2-(1-pyrrolidinyl)cyclohexanecarboxhydrazide hydrochloride in 100 ml of water, prepared as in part B hereinabove with ice cooling there was added 14.3 g (0.207 mole) of sodium nitrite in 25 ml of water over 30 minutes, while stirring. After 30 minutes 30 ml of 12N hydrochloric acid was added to the reaction mixture and the solution was slowly heated to 70°C on a water bath. When evolution of nitrogen by-product had nearly ceased, the solution was heated on a steam bath for 1 hour. The resulting solution was cooled, made basic with 15 percent sodium hydroxide solution, and extracted with ether. The ether extract was dried ($MgSO_4$) and evaporated to remove solvent. Distillation of the residue gave 25.7 g (81 percent yield) of the diamine, cis-2-(1-pyrrolidinyl)cyclohexaneamine, b.p. 120° - 123°C/14 mm (Hg). The UV, IR, NMR and Mass spectra were consistent with this named compound.

Anal. Calcd. for $C_{10}H_{20}N_2$:

| | |
|---|---|
| % Calcd.: | C, 71.37; H, 11.98; N, 16.65 |
| Found: | C, 71.05; H, 11.91; N, 16.49 |

D. N-Methyl-Cis-2-(1-Pyrrolidinyl)cyclohexylamine

A solution of 20.0 g (0.119 mole) of the cis-2-(1-pyrrolidinyl)cvclohexaneamine, from part C hereinabove in 250 ml of ethyl formate solvent was refluxed overnight. The solution was evaporated to remove solvent and leave a crude amide intermediate as a colorless oil product. The crude product was dissolved in 100 ml of tetrahydrofuran (THF) and added dropwise in one hour to a suspension of 20.0 g of lithium aluminum hydride in 1000 ml of ether. The mixture was refluxed for 6 hours and then kept overnight at room temperature. The mixture was cooled in ice and excess lithium aluminum hydride was decomposed by adding 20 ml of water, 20 ml of 15 percent sodium hydroxide aqueous solution and 60 ml of water. The mixture was filtered and the filtered by-product aluminum salt solids were washed with ether. The filtrate and ether washings were dried ($MgSO_4$) and evaporated to remove solvents. Distillation of the residue gave 16.5 g (76 percent yield) of the cis-N-methyl-N-[2-(1-pyrrolidinyl)cyclohexaneamine, b.p. 120° - 122°C/14 mm (Hg). The UV, IR, NMR and Mass

Spectra were consistent with this named compound.
Anal. Calcd. for $C_{11}H_{22}N_2$:

| | |
|---|---|
| % Calcd: | C, 72.46; H. 12.17; N, 15.37 |
| Found: | C, 72.64; H, 12.73; N, 15.42 |

**Example 1**

Cis-N-[2-(N',N'-dimethylamino)cyclohexyl]benzamide

To a mixture of 1.42 g (0.01 mole) of cis-[2-(dimethylamino)]cyclohexylamine (prepared as described in U.S. Patent No. 4 098 904 starting at column 21, line 67) and 1.01 g (0.01 mole) of triethylamine in 100 ml, of diethyl-ether there was added 1.41 g (0.01 mole) of benzoyl chloride in 25 ml of ether over 30 minutes with ice cooling of the reaction vessel. The reaction mixture was stirred overnight at room temperature. Then, 100 ml of a saturated sodium bicarbonate in water solution was added to the resulting reaction mixture. The aqueous layer was separated from the organic liquid layer. The organic liquid layer was then washed with brine (saturated aqueous sodium chloride), separated from the aqueous brine layers and dried using anhydrous magnesium sulfate and then evaporated to remove solvent and to leave a yellow oil residue, which residue became solid on standing, and which weighed 2.43 g (99 percent, crude yield) of the cis-N-[2-(dimethylamino)-cyclohexyl]-N-benzamide, m.p. 73° - 76°C.

The hydrochloride salt of this cis-N-[2-(dimethylamino)cyclohexyl]benzamide was made by dissolving this amino-amide compound in excess hydrogen chloride in ether solution, and then evaporating solvent therefrom to crystallize the hydrochloride salt therefrom. This amino-amide hydrochloride salt was recrystallized from a mixture of 10 ml of methanol and about 40 ml of ether to obtain 2.13 g (75 percent yield) of the cis-N-[2-(dimethylamino)cyclohexyl]benzamide hydrochloride, m.p: 247° - 248°C.

The elemental analysis for this amino-amide salt was:

Anal. Calcd. for $C_{15}H_{22}N_2O \cdot HCl$(282.81)

| | |
|---|---|
| % Calcd.: | C, 63.70; H, 8.20; N, 9.91; Cl, 12.54 |
| Found: | C, 63.90; H, 8.53; N, 9.92; Cl, 12.69 |

The Nuclear Magnetic Resonance (NMR) Spectrum ($D_2O$) was consistent for this named product. The Infrared (IR) spectrum was also consistent. The Ultraviolet (UV) light spectrum showed (ethyl alcohol) end absorption 227 (11,650), 226 sh (846), 269 (769). The Mass Spectrum showed a main ion of $M^+$246.

Following procedures known in the art or as described hereinabove the following additional examples of these cis-amino-amide compounds were prepared:

**Example 2**

Cis-3,4-dichloro-N-[2-(1-pyrrolidinyl)cyclopentyl]benzamide, and its 4-methylbenzenesulfonate salt, as its monohydrate

| **M.P.** | Anal. Calcd. for $C_{16}H_{20}Cl_2N_2O \cdot C_7H_8SO_3 \cdot H_2O$ |
|---|---|
| 215° - 216°C. | % Calcd.: C, 55.08; H, 6.03; N, 5.59; Cl, 14.14; S, 6.40 |
| (methanol/ether) | % Found: C, 55.08; H, 5.69; N, 5.63; Cl, 14.34, S, 6.53 |

**Example 3**

Cis-3,4-dichloro-N-methyl-N-[2-(1-pyrrolidinyl)cyclopentyl]benzamide, and its monohydrochloride salt

| **M.P** | Anal. Calcd. for $C_{17}H_{22}Cl_2N_2O$ |
|---|---|
| 245 - 247°C. | % Calcd.: C, 54.05; H, 6.14; N, 7.42; Cl, 28.16 |
| (methanol/ether) | % Found: C, 54.20; H, 6.08; N, 7.41; Cl, 28.31 |

**Example 4**

Cis-3,4-dichloro-N-[2-(1-pyrrolidinyl)cyclopentyl]benzeneacetamide

| **M.P.** | Anal. Calcd. for $C_{17}H_{22}Cl_2N_2O$ |
|---|---|

| | |
|---|---|
| 106°C. | % Calcd.: C, 59.82; H. 6.50; N, 8.21; Cl, 20.78 |
| (ether) | % Found : C, 59.94; H, 6.51; N, 8.21; Cl, 20.66 |

**Example 5**

Cis-3,4-dichloro-N-methyl-N-[2-(1-pyrrolidinyl)cyclohexyl]benzeneacetamide

| **M.P** | Anal. Calcd. for $C_{19}H_{26}Cl2N_2O$ |
|---|---|
| None (oil) | % Calcd.: C, 61.78; H, 7.10; N, 7.59; Cl, 19.20 |
| | % Found: C, 61.39; H, 7.15; N, 7.55; Cl, 18.51 |

**Example 6**

Cis-3,4-dichloro-N-[2-(1-pyrrolidinyl)cyclohexyl]benzamide, and its hydrochloride salt

| **M.P.** | Anal. Calcd. for $C_{17}H_{22}Cl_2N_2O{\cdot}HCl$ HCl |
|---|---|
| 265 - 267°C. | % Calcd.: C, 54.05; H, 6.14; N, 7.42; Cl, 28.16 |
| (methanol/ether) | % Found: C, 54.36; H, 6.30; N, 7.41, Cl, 28.16 |

**Example 7**

Cis-3,4-dichloro-N-methyl-N-[2-(1-pyrrolidinyl)cyclohexyl]benzamide and its monohydrochloride

| **M.P.** | Anal. Calcd. for $C_{18}H_{24}Cl_2N_2O{\cdot}HCl$ |
|---|---|
| 229°C. | |
| (methanol/ether) | % Calcd.: C, 55.18; N, 6.43; N, 7.18; Cl, 27.15 |
| | % Found : C, 55.43; H, 6.50; N, 7.18, Cl, 27.49 |

**Example 8**

Cis-3,4-dichloro-N-[2-(dimethylamino)cyclohexyl]benzeneacetamide

| **M.P.** | Anal. Calcd. for $C_{16}H_{22}Cl_2N_2O$ |
|---|---|
| 111° - 112°C. | % Calcd.: C, 58.36; H, 6.74; N, 8.51; Cl, 21.54 |
| (ether) | % Found : C, 58.70; H, 6.75; N, 8.51; Cl, 21.39 |

**Example 9**

Cis-3,4-dichloro-N-[2-(dimethylamino)cyclohexyl]benzamide, and its monohydrochloride

| **M.P.** | Anal. Calcd. for $C_{15}H_{20}Cl_2N_2O{\cdot}HCl$ |
|---|---|
| 231° - 232°C. | |
| (methanol/ether) | % Calcd.: C, 51.22; H, 6.02; N, 7.97, Cl, 30.24 |
| | % Found : C, 51.59; H, 6.15; N, 7.84; Cl, 30.09 |

**Example 10**

Cis-3,4-dichloro-N-methyl-N-[2-(dimethylamino)cyclohexyl]benzeneacetamide and its p-toluenesulfonate salt

| **M.P.** | Anal. Calcd. for $C_{17}H_{24}Cl_2N_2O{\cdot}C_7H_8SO_3$ |
|---|---|
| 175° - 176°C. | % Calcd.: C, 55.91; H, 6.26; N, 5.44; Cl, 13.76; S, 6.22 |
| (methanol/ether) | % Calcd.: C, 55.89; H, 6.42; N, 5.69, Cl, 13.81, S, 6.51 |

**Example 11**

Cis-4-(trifluoromethyl)-N[2-(dimethylamino)cyclohexyl]benzamide, and its monohydrochloride

| | |
|---|---|
| **M.P.** | Anal. Calcd. for $C_{16}H_{21}N_2F_3O{\cdot}HCl$ |
| 183° - 184°C. | % Calcd.: C, 54.77; H, 6.32; N, 7.99; F, 16.25; |
| (methanol/ether) | Cl, 10.11 |
| | % Found : C, 54.57; H, 6.43, N, 7.94; Cl, 10.08, |
| | F, 16.37 |

**Example 12**

Cis-3,4-dichloro-N-[2-(1-pyrrolidinyl)cyclohexyl]benzeneacetamide

| | |
|---|---|
| **M.P.** | Anal. Calcd. for $C_{18}H_{24}ClN_2O$ |
| 133° - 134°C. | % Calcd.: C, 60.79; H. 6, 68, N, 7.89; Cl, 19.96 |
| | % Found : C, 60.79; H, 6.68, N. 7.89, Cl, 20.18 |

**Example 13**

Anticonvulsant Use Test Results - Electroshock Test

This example exemplifies anticonvulsant activity properties of representative compounds of this invention in a standard laboratory animal electroshock test.

Male CF-1 mice were injected subcutaneously with the test compound, and after the compound was absorbed, the test mice were subjected to maximal transpinnal electroshock (10mA, 0.2 sec ) Protection from Central Nervous System (CNS) seizure by the test compound was judged by the absence of the tonic extensor convulsion. Each test drug substance or compound was formulated into 0.25 percent w/v aqueous methylcellulose solution and administered at a volume dose of 10 ml per kilogram of body weight. A group of six mice was treated and tested at each dose level, and decreasing doses (0.3 log intervals) were administered to such groups of mice until response activity was low enough so that $ED_{50}$ dose calculations within 95 percent confidence intervals could be established. Most of these exemplified compounds lack any significant analgesic activity ($ED_{50}$ greater than 50 in standard HCl writing analgesic test).

**Table 1**

**Electroshock Test Results**

($ED_{50}$, subcutaneous route, mg/kg) (95 percent, Confidence Index Range)

| Example No. Compound | Electroshock ($ED_{50}$, mg/kg) |
|---|---|
| 3 | 32 |
| 4 | 50 |
| 6 | 18 |
| 7 | 13 (9 - 17) |
| 8 | 50 (31 - 81) |
| 9 | 31 |
| 10 | 40 |
| 11 | 25 (17 - 30) |
| 12 | 62 |

**Example 14**

One thousand tablets for oral use, each containing 175 mg of cis-3,4-dichloro-N-methyl-N-[2-(1-pyrrolidinyl)cyclohexyl]benzamide hydrochloride as the essential active ingredient are prepared from the following ingredients:

Essential active ingredient: 175 gm
- Methylcellulose, U.S.P. (15 cps.) - 6.5 gm
- Talc - 20 gm
- Calcium Stearate - 2.0 gm

The essential active ingredient and dicalcium phosphate are mixed well, granulated with 7.5 % aqueous solution of methylcellulose, passed through a No. 8 screen and dried carefully. The dried granules are passed through No. 12 screen, mixed with the talc and stearate and compressed into tablets. These tablets are useful in the treatment of adult humans at a dose of 1 tablet to 4 times a day as needed to prevent convulsions.

**Example 15**

One thousand two-piece hard gelatin capsules for oral use, each capsule containing 100 mg of cis-3,4-dichloro-N-methyl-N-[2-(1-pyrrolidinyl)cyclohexyl]benzamide hydrochloride as the essential active ingredient are prepared from the following ingredients:

Essential active ingredient - 100 gm
Lactose, U.S.P. - 100 gm
Starch, U.S.P. - 10 gm
Talc, U.S.P. - 5 gm
Calcium Stearate - 1 gm
The finely powdered materials are mixed thoroughly, then filled into hard gelatin capsules of appropriate size.

One capsule four times a day is useful for the treatment of adult humans to block the occurrence of CNS convulsive seizures.

**Example 16**

A sterile aqueous suspension suitable for intramuscular injection and containing in each milliliter 50 mg of the Example 16 essential active ingredient is prepared from the following ingredients:

Essential active ingredient - 5 gm
Polyethylene glycol 4000, U.S.P. - 3 gm
Sodium chloride - 0.9 gm
Polysorbate 80, U.S.P. - 0.4 gm
Sodium metabisulfite - 0.1 gm
Methylparaben, U.S.P. - 0.18 gm
Propylparaben, U.S.P. - 0.02 gm
Water for injection, q.s. to - 100 ml.

The preceding sterile injectable is useful in the treatment of CNS convulsions in adult humans at a dose of 1/2 to 2 ml.

**General Formulas**

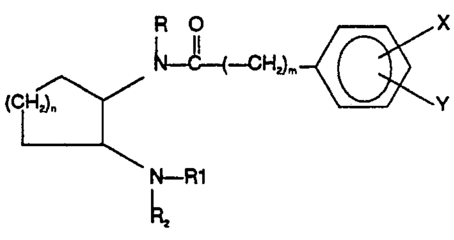

(I)

**Scheme I**

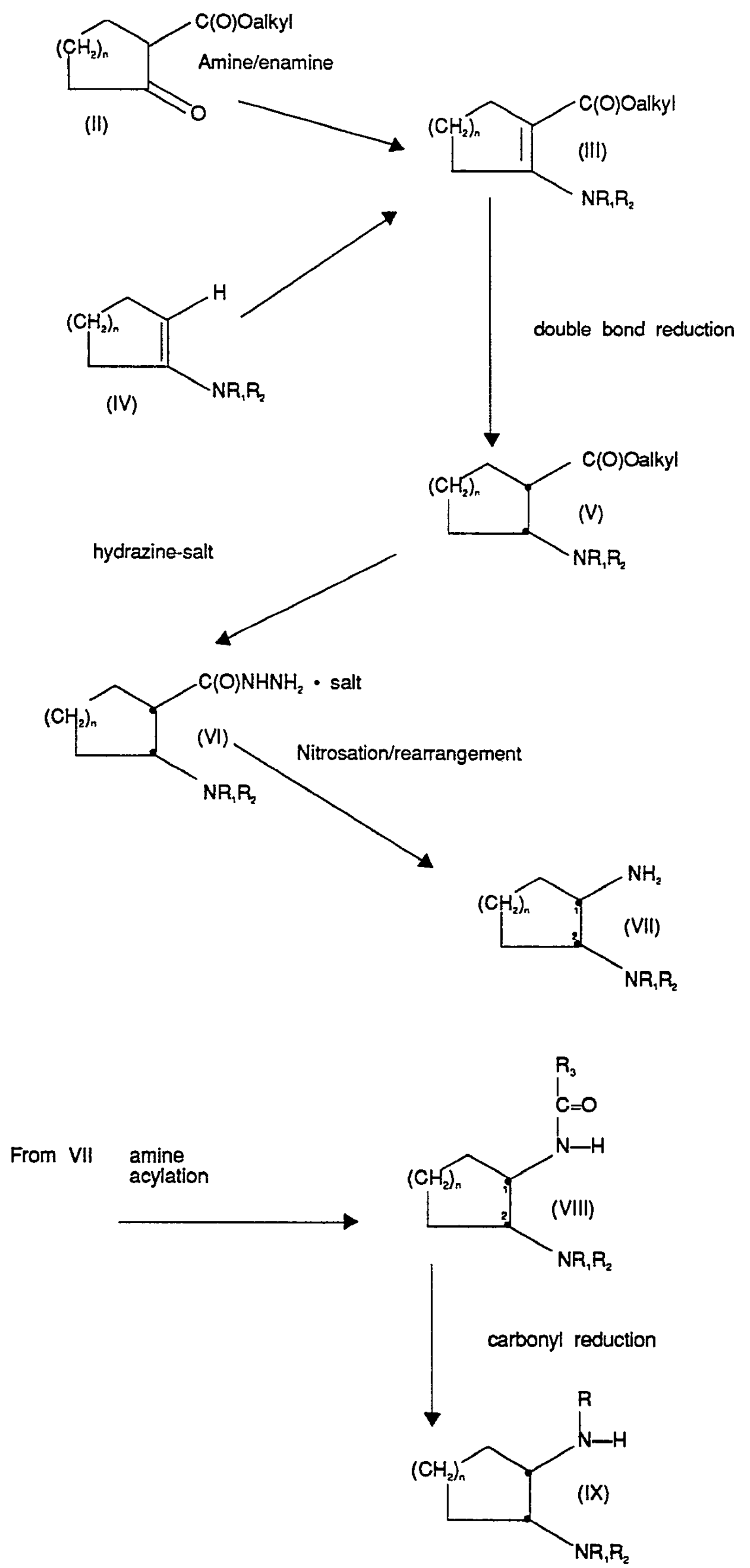
Amine/enamine
(II)
(III)
(IV)
double bond reduction
(V)
hydrazine-salt
(VI)
Nitrosation/rearrangement
(VII)
From VII amine acylation
(VIII)
carbonyl reduction
(IX)

**Scheme II**

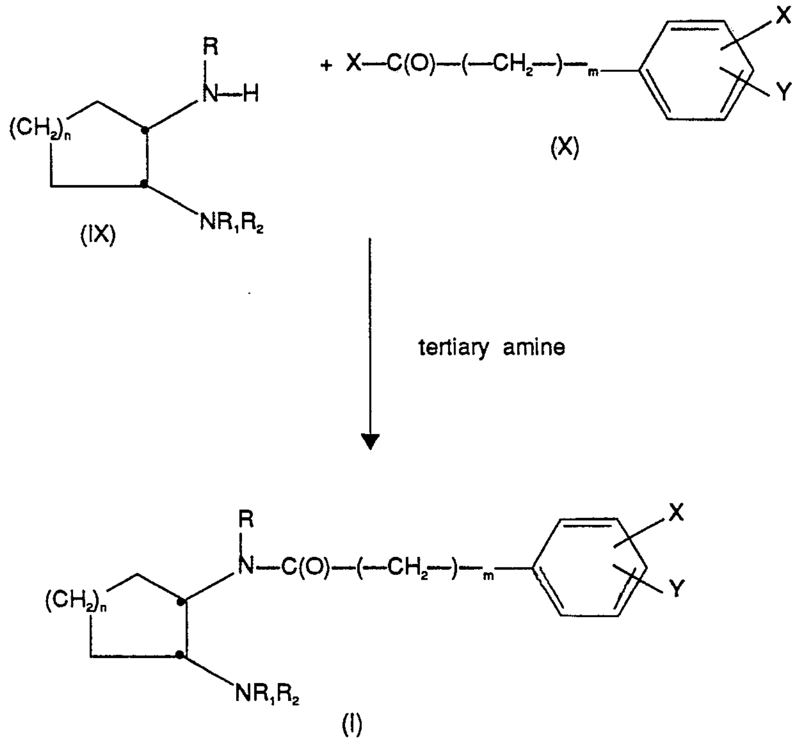


(IX) (X)

(I)

**Claims**

1. Use of a compound for the manufacture of a medicament for preventing or treating central nervous system seizures, in which the compound is of formula I

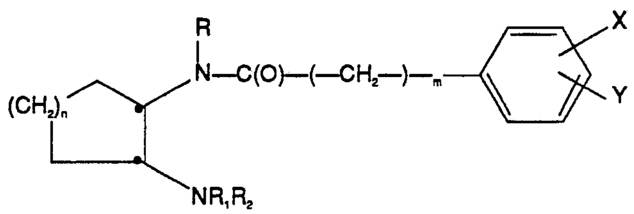

(I)

wherein the filled circles indicate that the two nitrogen atoms are in the cis orientation;

n is 1 or 2;
m is 0 or 1;
R is hydrogen or $C_{1-3}$ alkyl;

either $R_1$ and $R_2$ are the same or different $C_{1-3}$ alkyl groups, or $NR_1R_2$ is 1-pyrrolidinyl; and
either X and Y are independently selected from H, F, Br and Cl, or X is H and Y is $CF_3$;
or is a pharmaceutically-acceptable salt thereof.

2. Use according to claim 1, wherein the compound is selected from
cis-N-[2-(dimethylamino)cyclohexyl]benzamide,

cis-3,4-dichloro-N-methyl-N-[2-(1-pyrrolidinyl)cyclopentyl]benzamide,
cis-3,4-dichloro-N-methyl-N-[2-(1-pyrrolidinyl)cyclohexyl]benzeneacetamide,
cis-3,4-dichloro-N-[2-(1-pyrrolidinyl)-cyclohexyl]benzamide,
cis-3,4-dichloro-N-methyl-N-[2-(1-pyrrolidinyl)cyclohexyl]benzamide,
cis-3,4-dichloro-N-2[-(dimethylamino)cyclohexyl]benzeneacetamide,
cis-3,4-dichloro-N-[2-(dimethylamino)cyclohexyl]benzamide,
cis-4-trifluoromethyl-N-[2-(dimethylamino)cyclohexyl]benzamide,
cis-3,4-dichloro-N-[2-(1-pyrrolidinyl)cyclohexyl]benzeneacetamide,
and pharmacologically-acceptable salts thereof.

3. Use according to claim 2, wherein the compound is cis-3,4-dichloro-N-methyl-N-[2-(1-pyrrolidinyl)cyclohexyl]benzamide, cis-3, 4-dichloro-N-[2-(1-pyrrolidinyl)cyclohexyl]benzamide, or a pharmaceutically-acceptable salt of either compound.

4. Use according to claim 3, wherein the compound is cis-3,4-dichloro-N-[2-(1-pyrrolidinyl)cyclohexyl]benzamide hydrochloride or a pharmaceutically-acceptable salt thereof.

5. Use according to claim 3, wherein the compound is cis-3,4-dichloro-N-methyl-N-[2-(1-pyrrolidinyl)cyclohexyl]benzamide hydrochloride or a pharmaceutically-acceptable salt thereof.

6. A compound selected from
cis-3,4-dichloro-N-[2-(1-pyrrolidinyl)cyclopentyl]benzeneacetamide,
cis-3,4-dichloro-N-methyl-N-[2-(1-pyrrolidinyl)cyclopentyl]benzamide,
cis-3,4-dichloro-N-[2-(1-pyrrolidinyl)cyclohexyl]benzamide,
cis-3,4-dichloro-N-methyl-N-[2-(1-pyrrolidinyl)cyclohexyl]benzamide,
and pharmaceutically-acceptable salts thereof.

7. A compound according to claim 6, which is cis-3,4-dichloro-N-[2-(1-pyrrolidinyl)cyclopentyl]benzeneacetamide or a pharmaceutically-acceptable salt thereof.

8. A compound according to claim 6, which is cis-3,4-dichloro-N-methyl-N-[2-(1-pyrrolidinyl)cyclopentyl]benzamide or a pharmaceutically-acceptable salt thereof.

9. A compound according to claim 6, which is cis-3,4-dichloro-N-[2-(1-pyrrolidinyl)cyclohexyl]benzamide or a pharmaceutically-acceptable salt thereof.

10. A compound according to claim 6, which is cis-3,4-dichloro-N-methyl-N-[2-(1-pyrrolidinyl)cyclohexyl]benzamide or a pharmaceutically acceptable salt thereof.

**Revendications**

1. Utilisation d'un composé pour la préparation d'un médicament destiné au traitement préventif ou curatif de crises du système nerveux central, dans laquelle le composé répond à la formule I

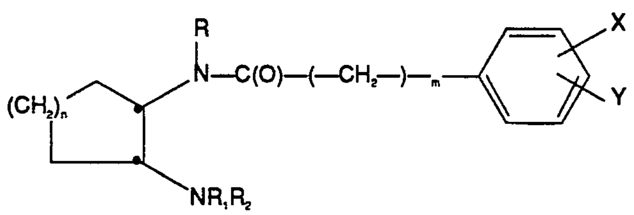

(I)

où les points renforcés signifient que les deux atomes d'azote ont l'orientation cis;

n a la valeur 1 ou 2 ;
m a la valeur 0 ou 1 ;
R est l'hydrogène ou un groupe alkyle en $C_1$ à $C_3$;
$R_1$ et $R_2$ sont des groupes alkyle en $C_1$ à $C_3$ identiques ou différents, ou bien $NR_1R_2$ représente un groupe 1-pyrrolidinyle; et

X et Y sont choisis indépendamment entre H, F, Br et Cl, ou bien X représente H et Y représente $CF_3$; ou est un sel pharmaceutiquement acceptable.

2. Utilisation suivant la revendication 1, dans laquelle le composé est choisi entre
le cis-N-[2-(diméthylamino)cyclohexyl]-benzamide,
le cis-3,4-dichloro-N-méthyl-N-[2-(2-pyrrolidinyl)cyclopentyl]benzamide,
le cis-3,4-dichloro-N-méthyl-N-[2-(1-pyrrolidinyl)cyclohexyl]benzène-acétamide,
le cis-3,4-dichloro-N-[2-(1-pyrrolidinyl)-cyclohexyl]benzamide,
le cis-3,4-dichloro-N-méthyl-N-[2-(1-pyrrolidinyl)-cyclohexyl]benzamide,
le cis-3,4-dichloro-N-[2-(diméthylamino)-cyclohexyl]benzène-acétamide,
le cis-3,4-dichloro-N-[2-(diméthylamino)-cyclohexyl]benzamide,
le cis-4-trifluorométhyl-N-[2-(diméthylamino)-cyclohexyl]benzamide,
le cis-3,4-dichloro-N-[2-(1-pyrrolidinyl)cyclohexyl]benzène-acétamide,
et leurs sels pharmacologiquement acceptables.

3. Utilisation suivant la revendication 2, dans laquelle le composé est le cis-3,4-dichloro-N-méthyl-N-[2-(1-pyrrolidinyl)cyclohexyl]benzamide, le cis-3,4-dichloro-N-[2-(1-pyrrolidinyl)cyclohexyl]benzamide, ou un sel pharmaceutiquement acceptable de l'un ou l'autre de ces composés.

4. Utilisation suivant la revendication 3, dans laquelle le composé est le chlorhydrate de cis-3,4-dichloro-N-[2-(1-pyrrolidinyl)cyclohexyl]benzamide ou un sel pharmaceutiquement acceptable de ce composé.

5. Utilisation suivant la revendication 3, dans laquelle le composé est le chlorhydrate de cis-3,4-dichloro-N-méthyl-N-[2-(1-pyrrolidinyl)cyclohexyl]benzamide ou un sel pharmaceutiquement acceptable de ce composé.

6. Composé choisi entre
le cis-3,4-dichloro-N-[2-(1-pyrrolidinyl)cyclopentyl]benzène-acétamide,
le cis-3,4-dichloro-N-méthyl-N-[2-(1-pyrrolidinyl)-cyclopentyl]benzamide,
le cis-3,4-dichloro-N-[2-(1-pyrrolidinyl)cyclohexyl]benzamide,
le cis-3,4-dichloro-N-méthyl-N-[2-(1-pyrrolidinyl)-cyclohexyl]benzamide,
et leurs sels pharmaceutiquement acceptables.

7. Composé suivant la revendication 6, qui est le cis-3,4-dichloro-N-[2-(1-pyrrolidinyl)cyclopentyl]benzène-acétamide ou un sel pharmaceutiquement acceptable de ce composé.

8. Composé suivant la revendication 6, qui est le cis-3,4-dichloro-N-méthyl-N-[2-(1-pyrrolidinyl)cyclopentyl]benzamide ou un sel pharmaceutiquement acceptable de ce composé.

9. Composé suivant la revendication 6, qui est le cis-3,4-dichloro-N-[2-(1-pyrrolidinyl)cyclohexyl]benzamide ou un sel pharmaceutiquement acceptable de ce composé.

10. Composé suivant la revendication 6, qui est le cis-3,4-dichloro-N-méthyl-N-[2-(1-pyrrolidinyl)cyclohexyl]benzamide ou un sel pharmaceutiquement acceptable de ce composé.

**Patentansprüche**

1. Verwendung einer Verbindung zur Herstellung eines Arzneimittels zur Vorbeugung oder Behandlung von Anfällen des Zentralnervensystems, wobei die Verbindung die Formel I

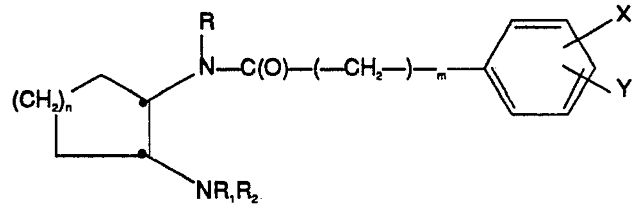

(I)

aufweist, worin die ausgefüllten Punkte anzeigen, daß die zwei Stickstoffatome in cis-Stellung stehen;

n 1 oder 2 bedeutet,
m 0 oder 1 bedeutet,
R Wasserstoff oder $C_{1-3}$ Alkyl bedeutet,
$R_1$ und $R_2$ entweder gleiche oder voneinander verschiedene $C_{1-3}$ Alkylgruppen bedeuten oder $NR_1R_2$ 1-Pyrrolidinyl bedeutet und
X und Y entweder unabhängig voneinander aus H, F, Br und Cl ausgewählt sind oder X Wasserstoff und Y $CF_3$ bedeutet,
oder ein pharmazeutisch verträgliches Salz davon.

2. Verwendung nach Anspruch 1 worin die Verbindung aus
cis-N-[2-(Dimethylamino)cyclohexyl]benzamid,
cis-3,4-Dichlor-N-methyl-N-[2-(1-pyrrolidinyl)-cyclopentyl]benzamid,
cis-3,4-Dichlor-N-methyl-N-[2-(1-pyrrolidinyl)-cyclohexyl]benzolacetamid,
cis-3,4-Dichlor-N-[2-(1-pyrrolidinyl)-cyclohexyl]benzamid,
cis-3,4-Dichlor-N-methyl-N-[2-(1-pyrrolidinyl)-cyclohexyl]benzamid,
cis-3,4-Dichlor-N-2-[-(dimethylamino)cyclohexyl]benzolacetamid,
cis-3,4-Dichlor-N-[2-(dimethylamino)cyclohexyl]benzamid,
cis-4-Trifluormethyl-N-[2-(dimethylamino)cyclohexyl]benzamid,
cis-3,4-Dichlor-N-[2-(1-pyrrolidinyl)cyclohexyl]benzolacetamid,
und pharmakologisch verträglichen Salzen davon ausgewählt ist.

3. Verwendung nach Anspruch 2, worin die Verbindung
cis-3,4-Dichlor-N-methyl-N-[2-(1-pyrrolidinyl)cyclohexyl]benzamid,
cis-3,4-Dichlor-N-[2-(1-pyrrolidinyl)cyclohexyl/benzamid, oder ein pharmazeutisch verträgliches Salz davon ist.

4. Verwendung nach Anspruch 3, worin die Verbindung cis-3,4-Dichlor-N-[2-(1-pyrrolidinyl)cyclohexyl]benzamid. Hydrochlorid oder ein pharmazeutisch verträgliches Salz davon ist.

5. Verwendung nach Anspruch 3, worin die Verbindung
cis-3,4-Dichlor-N-methyl-N-[2-(1-pyrrolidinyl)cyclohexyl]benzamid. Hydrochlorid oder ein pharmazeutisch verträgliches Salz davon ist.

6. Verbindung gewählt aus
cis-3,4-Dichlor-N-[2-(1-pyrrolidinyl)cyclopentyl]benzolactetamid,
cis-3,4-Dichlor-N-methyl-N-[2-(1-pyrrolidinyl)cyclopentyl]benzamid,
cis-3,4-Dichlor-N-[2-(1-pyrrolidinyl)cyclohexyl]benzamid,
cis-3,4-Dichlor-N-methyl-N-[2-(1-pyrrolidinyl)cyclohexyl]benzamid
oder einem pharmazeutisch verträglichen Salz davon.

7. Verbindung nach Anspruch 6, welche
cis-3,4-Dichlor-N-[2-(1-pyrrolidinyl)cyclopentyl]benzolacetamid oder ein pharmazeutisch verträgliches Salz davon ist.

8. Verbindung nach Anspruch 6, welche
cis-3,4-Dichlor-N-methyl-N-[2-(1-pyrrolidinyl)cyclopentyl]benzamid oder ein pharmazeutisch verträgliches Salz davon ist.

9. Verbindung nach Anspruch 6, welche
cis-3,4-Dichlor-N-[2-(1-pyrrolidinyl)cyclohexyl]benzamid oder ein pharmazeutisch verträgliches Salz davon ist.

10. Verbindung nach Anspruch 6, welche
cis-3,4-Dichlor-N-methyl-N-[2-(1-pyrrolidinyl)cyclohexyl]benzamid oder ein pharmazeutisch verträgliches Salz davon ist.